Europäisches Patentamt

European Patent Office

Office européen des brevets

(19)

(11) Publication number: 0 189 250 B1

# EUROPEAN PATENT SPECIFICATION

(12)

(45) Date of publication of patent specification: **09.09.92**    (51) Int. Cl.⁵: **A61B 5/04**

(21) Application number: **86300105.3**

(22) Date of filing: **08.01.86**

(54) **Electrode securement sheet.**

(30) Priority: **22.01.85 JP 6901/85**

(43) Date of publication of application:
**30.07.86 Bulletin 86/31**

(45) Publication of the grant of the patent:
**09.09.92 Bulletin 92/37**

(84) Designated Contracting States:
**DE FR GB IT SE**

(56) References cited:
**US-A- 3 545 432**
**US-A- 3 565 059**
**US-A- 4 050 453**
**US-A- 4 331 153**

(73) Proprietor: **FUKUDA DENSHI CO., LTD.**
**39-4, Hongo 3-chome Bunkyo-ku**
**Tokyo 113(JP)**

(72) Inventor: **Inoue, Hirokatsu**
**1800-142, Kasoricho**
**Chiba City Chiba Pref.(JP)**
Inventor: **Shimizu, Chuji**
**No. 2-2-3 Narashinodai**
**Funabashi City Chiba Pref.(JP)**

(74) Representative: **Stuart, Ian Alexander et al**
**MEWBURN ELLIS & CO. 2/3 Cursitor Street**
**London EC4A 1BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Description

This invention relates to an electrode securement sheet for securing an electrode in close contact with the skin of a living body to the skin surface. Cross-reference is made to EP-A-0189251.

As is well known in the art, electricity is induced in the living body by the activity of the heart, brain, muscles, etc.

Particularly, electricity produced by the activity of the heart is detected as a weak current induced on the skin surface of the living body using an external electrocardiograph to check for abnormality of the heart. To this end, electrodes of an input section of the electrocardiograph are held in close contact with the skin surface of the living body and thereby electrically coupled thereto.

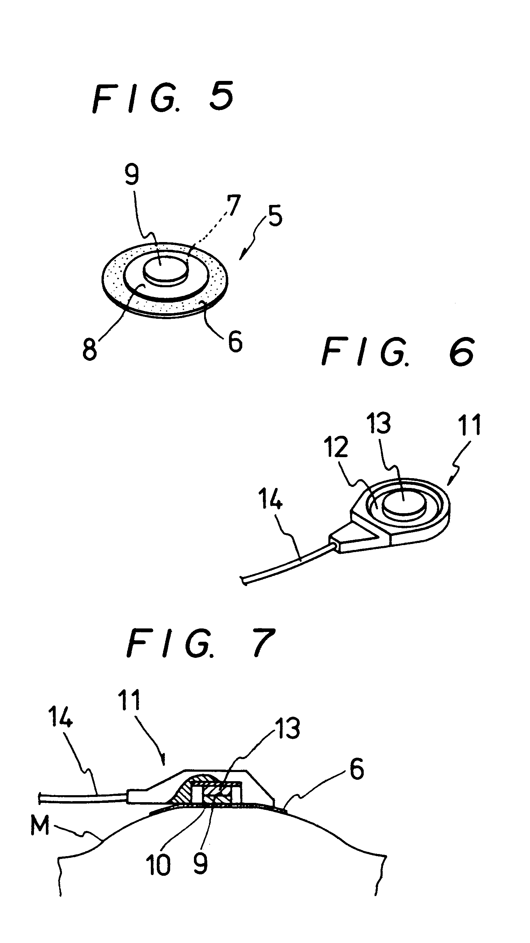

Figs. 5 to 7 illustrate a prior art current derivation electrode that is held in close contact with the skin surface of a living body. Fig. 5 shows the electrode 5 in perspective. The electrode 5 is provided with a substantially circular, sticky sheet piece 6. The sticky sheet piece 6 has a doughnut-like cloth piece with a central opening 7. Its lower surface is sticky enough to be held in close contact with the skin surface M of the living body, as shown in Fig. 7.

An electrode plate engagement member 8 made of a hard synthetic resin is bonded to the upper surface of the sticky sheet piece 6 to close the opening 7. The electrode plate engagement member 8 has a magnetic lead coupler 9 projecting from the upper surface. As shown in Fig. 7, an electrode plate 10, which is to be held in close contact with the skin surface M of the living body to derive a weak current from the heart, is secured to the lower surface of the lead coupler 9.

Fig. 6 is a back view of a lead connector 11, through which a weak current derived from the heart through the electrode plate 10 is led via a lead to an electrocardiograph installed in a room. The lead connector 11 has substantially the same size as the electrode 5 and is made of a hard resin. It has a recess 12, and a magnetic electrode coupler 13 is provided in the recess 12 and secured to the lead connector 11. One end of a lead 14 is connected to the electrode coupler 13, and its other end is connected to the electrocardiograph (not shown).

To obtain an electrocardiograph using the current derivation electrode 5 as described above, the sticky sheet piece 6 of the electrode 5 is first applied to the skin surface M of a living body, as shown in Fig. 7, and then the lead connector 11 is coupled to the electrode 5 by bonding the magnetic electrode coupler 13 of the lead connector 11 to the lead coupler 9 of the electrode 5. In this state, a weal voltage or current from the heart, derived through the electrode plate 10, is led through the lead 14 to the electrograph.

Usually, a weak current derived from the skin of a patient with a serious cardiac disease in a ward of a hospital through the electrode 5 held in close contact with the patient's skin surface, is led to an electrocardiograph installed in a separate serious disease patient watch-over room to be checked by the watch-over personnel.

However, the lead connector 11 that is coupled to the electrode 5 held in close contact with the patient's skin surface, is liable to be shifted from a proper position with respect to the electrode 5 due to an unconcious movement of the patient such as tossing-about in sleep. In such a case, noise is produced, so that an accurate electrocardiogram can not be obtained.

Further, it is liable that the patient unconciously pulls out the lead 14 of the lead connector 11. In such a case, the lead connector 11 is liable to be detached from the electrode 5. When the lead connector 11 is detached, an alarm device provided in the serious disease patient watch-over room is driven. When this happens, the watch-over personnel has to hasten to the ward to check the patient's condition.

The alarm device is designed to produce an alarm in such an emergency case as when the pulsation of the heart of a patient is stopped during recording of the patient's electrocardiogram with the electrode 5 held in close contact with the patient's skin surface. When the lead connector 11 or electrode 5 is detached from the patient's skin, the alarm device is also driven in spite of the fact that the patient's heart is normal. Even in such a case, the watch-over personnel has to hasten from the serious disease patient watch-over room to the patient's ward. If the alarm device is erroneously driven frequently, the fatigue of the watch-over personnel is extremely increased.

Further, there is a case when an electrocardiogram of a patient during motion is recorded depending on the patient's condition. In this case, if a shift or detachment of the lead connector 11 coupled to the electrode in close contact with the patient's skin surface occurs due to a patient's motion, an accurate electrocardiogram can no longer be obtained.

Preferred embodiments of the invention solve the problems discussed above which are posed in the prior art.

The present invention provides an electrode securement sheet assembly as defined in claim 1.

An embodiment of the invention will now be described in greater detail with reference to the accompanying drawings in which:

Fig. 1 is a perspective view showing an embodiment of the electrode securement sheet according to the invention;

Fig. 2 is a plan view showing the electrode securement sheet shown in Fig. 1;

Fig. 3 is a sectional view taken along line III-III in Fig. 2;

Fig. 4 is a view showing the electrode securement sheet according to the invention in use;

Fig. 5 is a perspective view showing a prior art electrode for deriving current from a living body;

Fig. 6 is a back view showing a lead connector; and

Fig. 7 is a view for explaining the electrode and lead connector in use.

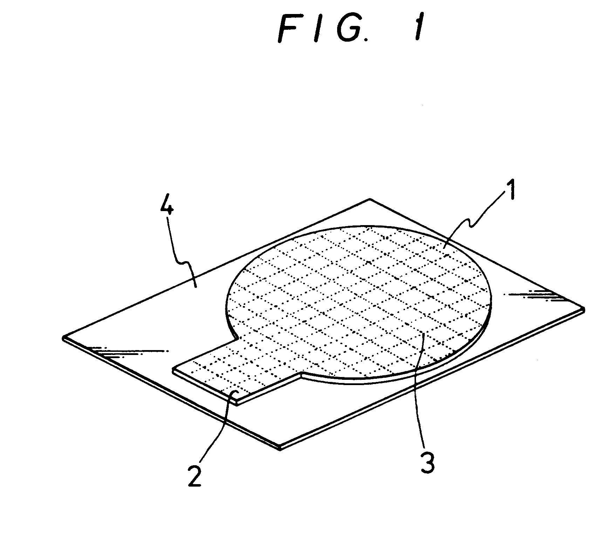

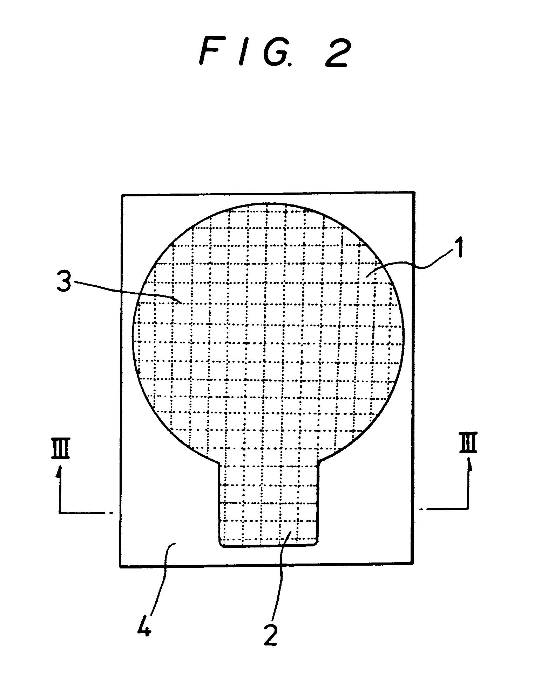

Fig. 1 is a perspective view showing an embodiment of the electrode securement sheet according to the invention, and Fig. 2 is a plan view of the same electrode securement sheet. Referring to the Figures, reference numeral 1 designates a substantially circular electrode securement section, which is adapted to be secured to the skin surface of a living body to cover the electrode 5 noted above, held in close contact with the skin surface of the living body to derive a weak current therefrom.

The electrode securement section 1 consists of a polyvinyl chloride sheet having a size sufficient to cover the electrode 5. It is porous, having numerous ventilating pores 3. This has an effect of preventing the electrode securement section 1 form being detached from the skin surface of the living body due to moistening of the section 1 caused by the action of sweating of the skin of the living body. The electrode securement section 1 is extensible, so that it can be readily attached to the skin surface by slightly spreading it. Also, it can be difficultly detached after it has once been attached.

The electrode securement section 1 has a rectangular lead securement portion 2 extending from one end. The lead securement portion 2 is made from the same porous and extensible polyvinyl chloride sheet. The electrode securement section 1 and lead securement section 2 may be made from a porous non-woven cloth or from a porous braided sheet as well as the porous polyvinyl chloride resin sheet.

Reference numeral 4 designates a thick cardboard. The electrode securement section 1 and lead securement portion 2 are separably bonded by an adhesive to the cardboard 4.

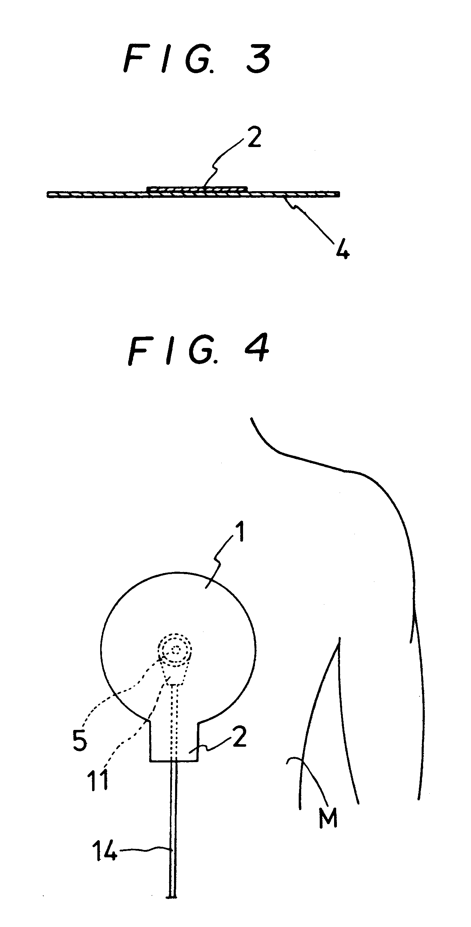

The electrode securement sheet having the above structure is used as follows. As shown in Fig. 4, the electrode 5 is set on the skin surface M of a man in close contact therewith. Then, the lead connector 11 is coupled to the top of the electrode 5. Thereafter, the sheet-like electrode securement section 1 with the lead securement portion 2 is separated from the cardboard 4 as shown in Figs. 1 and 2, and is applied to the skin surface M to cover the entire electrode 5, to which the lead connector 11 is coupled, as shown in Fig. 4. The electrode 5 thus can be secured to the skin surface M of the man by the electrode securement section 1 with the lead 14 covered by the lead securement portion 2.

As has been described in the foregoing, according to the invention the electrode for extracting a current from a living body is secured to the skin surface thereof by the electrode securement section with a lead securement portion. The electrode thus is firmly held in close contact with the skin of a man and will never be detached therefrom during examination. It is thus possible to eliminate erroneous operations of alarm device provided in serious disease patient watch-over room and reduce fatigue of the watch-over personnel.

Further, since the electrode is firmly held in close contact with the skin surface of a man by the electrode securement section with the lead securement portion, it will never be shifted or detached even in case of recording an electrocardiogram while the patient is in motion. Such an electrocardiogram thus can be readily obtained.

Further, since the electrode securement section and lead securement section are integral with each other, the electrode securement sheet according to the invention can be secured to the man's skin in a single operation. The electrode securement sheet thus can save time and has satisfactory operability.

Further, since the electrode securement section and lead securement section are porous, they will not be moistened by the action of the sweating of the skin when it is in close contact therewith, thus eliminating the possibility of its detachment from the skin due to moistening. Further, since the electrode securement sheet is extensible, it can be attached to the skin surface by slightly stretching it. It thus can be held in satisfactorily close contact with the skin surface.

## Claims

1. An electrode securement sheet assembly for use in securing to a patient an electrode assembly (5-14) of the type comprising an electrode portion (5) which is adapted to be adhered to a patient's skin and has an upstanding magnetic lead coupler (9), and a lead connector portion (11) for connection to the electrode portion (5), said connector portion (11) having a rigid body for overlying substantially the whole of the electrode portion (5), and a lead (14) extending from the body; the body having a recess (12) from the base of which a magnetic electrode coupler (13) projects so as to engage the magnetic lead coupler (9) when the connector portion (11) is overlying the electrode portion (5);

said electrode securement sheet assembly comprising a support (4) and a sheet (1,2) of porous extensible material, said sheet comprising a substantially circular electrode securement portion (1) and, integral therewith, a lead securement portion (2) extending from a portion of the periphery of the circular portion (1) said support (4) and sheet (1,2) being separably bonded together by an adhesive; the arrangement being such that, in use, when an electrode assembly (5-14) is adhered to a patient's skin it can be secured in place by separating the sheet (1,2) from the support (4) and applying it over the electrode assembly, stretching the sheet slightly so that it is brought into close contact with the skin around the electrode assembly, the lead securement portion (2) securing the lead (14) to the skin; the porous nature of the sheet allowing sweat to escape without dislodging the sheet.

2. An electrode securement sheet assembly according to claim 1, wherein said electrode securement portion (1) and lead securement portion (2) comprise a porous resin sheet.

3. An electrode securement sheet assembly according to claim 1, wherein said electrode securement portion (1) and lead securement portion (2) comprise a porous non-woven cloth.

4. An electrode securement sheet assembly according to claim 1, wherein said electrode securement portion (1) and lead securement portion (2) comprise a porous woven cloth.

5. An electrode securement sheet assembly according to any preceding claim wherein said support (4) comprises cardboard.

**Patentansprüche**

1. Blattförmige Elektrodenhalterung, um an einem Patienten eine Elektrodenbaugruppe (5-14) einer Bauart zu befestigen, umfassend einen Elektrodenbereich (5), der an der Haut eines Patienten anhaften kann und eine nach oben ragende magnetische Zuleitungskupplung (9) hat, und einen Zuleitungsverbindungsbereich (11) zur Verbindung mit dem Elektrodenbereich (5), wobei der Verbindungsbereich (11) einen starren Körper, der im wesentlichen den gesamten Elektrodenbereich (5) überdeckt, und eine sich von dem Körper erstreckende Zuleitung (14) aufweist; wobei der Körper eine Ausnehmung (12) aufweist, von deren Grundfläche eine magnetische Elektrodenkupplung (13) nach oben ragt, so daß sie mit der magnetischen Zuleitungskupplung (9) in Eingriff gelangt, wenn der Verbindungsbereich (11) den Elektrodenbereich (5) überdeckt; wobei die blattförmige Elektrodenhalterung eine Unterlage (4) und ein Blatt (1,2) aus einem porösen dehnbaren Material umfaßt, wobei das Blatt einen im wesentlichen kreisrunden Elektrodenhalterungsbereich (1) und einem damit einstückigen Zuleitungshalterungsbereich (2) aufweist, der sich von einem Umfangsbreich des kreisrunden Bereichs (1) erstreckt; wobei die Unterlage (4) und das Blatt (1,2) mit einem Klebstoff lösbar miteinander verklebt sind; wobei die Anordnung derart ist, daß im Gebrauch eine Elektrodenbaugruppe (5-14) an der Haut eines Patienten befestigt werden kann, indem das Blatt (1,2) von der Unterlage (4) getrennt und auf der Elektrodenbaugruppe angebracht wird, wobei das Blatt leicht gedehnt wird, um es mit der die Elektrodenbaugruppe umgebenden Haut in enge Berührung zu bringen, wobei der Zuleitungshalterungsbereich (2) die Zuleitung (14) an der Haut festlegt, wobei die Porösität des Blattes das Entweichen von Schweiß ermöglicht, ohne das Blatt zu verlagern.

2. Blattförmige Elektrodenhalterung nach Anspruch 1, wobei der Elektrodenhalterunsbereich (1) und der Zuleitungshalterungsbereich (2) aus einer porösen Kunststoffolie besteht.

3. Blattförmige Elektrodenhalterung nach Anspruch 1, wobei der Elektrodenhalterungsbereich (1) und der Zuleitungshalterungsbereich (2) aus einem porösen nicht-gewebten Textilerzeugnis besteht.

4. Blattförmige Elektrodenhalterung nach Anspruch 1, wobei der Elektrodenhalterungsbereich (1) und der Zuleitungshalterungsbereich (2) aus einem porösen gewebten Textilerzeugnis besteht.

5. Blattförmige Elektrodenhalterung nach einem der vorhergehenden Ansprüche, wobei die Unterlage (4) aus Karton besteht.

**Revendications**

1. Assemblage de feuille de fixation pour électrode, destiné à être utilisé pour fixer, à un patient, un assemblage formant électrode (5-14) du type comprenant une portion d'électrode (5) qui est apte à adhérer à la peau d'un patient et présente un coupleur magnétique de fil (9) debout et une portion de connecteur de fil (11) pour la connexion à la portion d'électrode (5), ladite portion de connecteur (11) possédant un corps rigide pour couvrir substantiellement toute la portion d'électrode (5), ainsi qu'un fil (14) s'étendant à partir du corps; le corps possédant un évidement (12) et de la base de celui-ci fait saillie un coupleur magnétique d'électrode (13) de façon à venir en prise avec le coupleur magnétique de fil (9) lorsque la portion de connecteur (11) recouvre la portion d'électrode (5);

ledit assemblage de feuille de fixation pour électrode comprenant un support (4) et une feuille (1, 2) en une matière poreuse extensible, ladite feuille comprenant une portion de fixation d'électrode (1) substantiellement circulaire et, réalisée en une pièce avec celle-ci, une portion de fixation de fil (2) s'étendant à partir d'une portion de la périphérie de la portion circulaire (1); lesdits support (4) et feuille (1,2) étant liés ensemble, de façon séparable, par un adhésif; l'agencement étant tel que, en cours d'utilisation, lorsqu'un assemblage d'électrode (5-14) est attaché à la peau d'un patient, il peut être fixé sur place en séparant la feuille (1,2) du support (4) et en l'appliquant sur l'assemblage formant électrode, en étirant légèrement la feuille de façon à l'amener en contact serré avec la peau autour de l'assemblage formant électrode, la portion de fixation de fil (2) fixant le fil (4) à la peau; la nature poreuse de la feuille permettant à la sueur de s'échapper sans déloger la feuille.

2. Assemblage de feuille de fixation pour électrode selon la revendication 1, dans lequel lesdites portion de fixation d'électrode (1) et portion de fixation de fil (2) comprennent une feuille poreuse en résine.

3. Assemblage de feuille de fixation pour électrode selon la revendication 1, dans lequel lesdites portion de fixation d'électrode (1) et portion de fixation de fil (2) comprennent une étoffe poreuse non tissée.

4. Assemblage de feuille de fixation pour électrode selon la revendication 1, dans lequel lesdites portion de fixation d'électrode (1) et portion de fixation de fil (2) comprennent une étoffe poreuse tissée.

5. Assemblage de feuille de fixation pour électrode selon l'une des revendications précédentes, dans lequel ledit support (4) comprend du carton.

# F I G. 1

# F I G. 2

# F I G. 3

# F I G. 4

# FIG. 5

# FIG. 6

# FIG. 7